# EUROPEAN PATENT APPLICATION

(11) **EP 4 721 763 A2**
(43) Date of publication of application: **08.04.2026**
(21) Application number: 26160058.9
(22) Date of filing: 08.06.2020
(51) Int. Cl.: A61K 47/36

(54) **IDENTIFICATION AND SELECTION OF A PLANT STARTING MATERIAL OF A PLANT CHONDROITIN SULFATE AND HYALURONIC ACID, AND TRANSFORMATION OF SUCH PLANT STARTING MATERIAL TO OBTAIN INGREDIENTS FOR USE IN FOODS, SUPPLEMENTS, MEDICAL DEVICES OR DRUGS**

(30) Priority: 07.06.2019 IT 201900008409
(62) Divisional of application: 20736429.0
(71) Applicant: Vivatis Pharma GmbH, 20097 Hamburg (DE)
(72) Inventor: The designation of the inventor has not yet been filed
(74) Representative: Marben S.r.l.

(57) **Abstract**

Process for extraction from a plant starting material, such as a fungus, for the preparation of a mixture (m) comprising or, alternatively, consisting of at least one glycosaminoglycan selected from: (a) hyaluronic acid or a salt thereof (HA) having a weight average molecular weight of comprised from 10 kDa to 600 kDa; (b) chondroitin or chondroitin sulfate or a salt thereof (CS) having a weight average molecular weight comprised from 3 kDa to 50 kDa; and (c) a combination of (a) and (b).

## Description

The present invention relates to a mixture comprising or, alternatively, consisting of at least one glycosaminoglycan, obtained from a plant starting material, selected from the group comprising or, alternatively, consisting of hyaluronic acid or a salt thereof (a hyaluronate anion salt) (in short, together or separate, HA), and/or chondroitin or a salt thereof such as a chondroitin sulfate or a salt thereof (in short, together or separate, CS), of plant origin having a high degree of purity and reduced content of contaminants and/or by-products.

Furthermore, the present invention relates to a use of the aforementioned mixture as an additive, excipient, or ingredient in the preparation of pharmaceutical products, medical devices, nutraceutical products, food for special medical purposes (FSMPs), food products or dietary supplements.

Furthermore, the present invention relates to a composition comprising (i) said mixture comprising or, alternatively, consisting of hyaluronic acid or a salt thereof (a hyaluronate anion salt) (in short, together or separate, HA), and/or chondroitin or a salt thereof, such as a chondroitin sulfate or a salt thereof, (in short, CS), of plant origin, and (ii) optionally technological additives and pharmaceutical or food grade excipients.

Furthermore, the present invention relates to said composition comprising said mixture for use as medicament.

Furthermore, the present invention relates to said composition comprising said mixture for use in a method for the preventive or curative treatment of humans and animals having specific disorders or pathologies or diseases, selected from arthritis, osteoarthritis, arthrosis, joint pain, inflammation of the limbs and joints, gastroesophageal reflux.

Furthermore, the present invention relates to a process for preparing said mixture, and said composition comprising said mixture, comprising or, alternatively, consisting of hyaluronic acid or a salt thereof (a hyaluronate anion salt) (in short, together or separate, HA), and/or a chondroitin or a salt thereof, such as a chondroitin sulfate or a salt thereof, (in short CS) of plant origin.

Lastly, the present invention relates to the use of a fungus as a plant starting material for preparing a hyaluronic acid or a salt thereof and/or a chondroitin or a salt thereof, such as a chondroitin sulfate or a salt thereof, of plant origin with a high degree of purity and a reduced content of contaminants and/or by-products.

Hyaluronic acid is an anionic non-sulfate glycosaminoglycan (GAG) which is distributed abundantly in connective, epithelial and neural tissues of vertebrates. Hyaluronic acid has important structural, rheological and physiological functions.

The rooster crests and human umbilical cords have very high concentrations of hyaluronic acid, respectively 7,500 mg/l and 4,100 mg/l. For this reason, in the early years '80, Endre A. Balazs and his collaborators developed a procedure for isolating and purifying hyaluronic acid from the rooster crests and from the human umbilical cords. Since then, hyaluronic acid has been produced from the rooster crests on an industrial scale.

Chondroitin sulfate is a sulfate GAG which consists of a chain of alternating units of sugar, N-acetylgalactosamine and glucuronic acid. A chondroitin sulfate chain can consist of hundreds of sugar units, each of which can be sulfated in varying positions and amounts. Due to its high compressive strength, chondroitin sulfate is an important structural component of the cartilage.

Chondroitin sulfate has a repeating unit (disaccharide) of the following general formula (I): 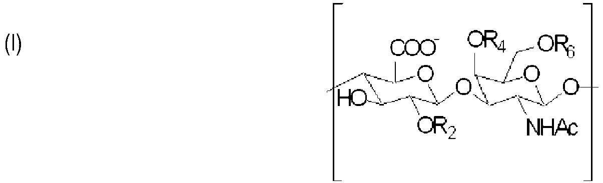 wherein at least one from among R₂, R₄ and R₆ is a sulfite group (S0₃⁻). In chondroitin mono-sulfate, only one of the R₂, R₄ or R₆ groups is a sulfite group. The three possible mono-sulfated chondroitins are therefore 6-chondroitin sulfate (R₂ = H; R₄ = H; R₆ = SO₃⁻) , 4-chondroitin sulfate (R₂ = H;R₄ = SO₃⁻; R₆ = H) and 2-chondroitin sulfate (R₂ = SO₃⁻; R₄ = H; R₆ = H).

Most chondroitin sulfate is obtained from extracts of animal cartilage, mainly from bovine and porcine tissues (for example: trachea, ear and nose), but other sources such as shark, fish and bird cartilage can also be used.

Despite the numerous and undisputed - in terms of efficacy - medical applications of hyaluronic acid and chondroitin sulfate in mammals, in particular in humans, the processes of preparation through extraction from animal precursors are today faced with growing concerns and fears, also of an ethical, religious and moral nature. The main concerns and fears arise from the use of products derived from animals or of animal origin for the preparation of a hyaluronic acid or a salt thereof and/or a chondroitin or a salt thereof, such as a chondroitin sulfate, especially when these compounds or salts are intended for nutritional, biomedical or pharmaceutical applications. In addition, chondroitin sulfate and hyaluronic acid extracted from animal sources have high molecular weights, while it would be advantageous to have a chondroitin sulfate and/or a hyaluronic acid with low molecular since they have better transcutaneous penetration properties.

In the prior art chondroitin sulfate, not of animal origin, obtained by inserting a sulfate group on a non-sulfated chondroitin obtained by means of bacterial fermentation processes, are known.

Therefore, in the field of pharmaceutical products, medical devices, nutraceutical products, food for special medical purposes (FSMPs), dietary supplements or food products, there is a strong need and demand by market operators to have a hyaluronic acid or a salt thereof and/or a chondroitin or a salt thereof, such as a chondroitin sulfate, that are prepared in an alternative way with respect to the existing ones and that can be used across all categories of consumers including vegans, vegetarians, subjects suffering from allergies and anyone who - for religious or ideological reasons - access to products or medicaments containing hyaluronic acid or a salt thereof and/or a chondroitin or salt thereof is currently precluded. Furthermore, the need is felt to produce chondroitin sulfate and/or hyaluronic acid of non-animal origin with processes that are economically advantageous with respect to what is known in the art and easy to apply.

After a long and intense research and development activity, the Applicant developed a preparation technology and process capable of providing an adequate response to the existing limits, drawbacks and problems.

Thus, forming an object of the present invention is a mixture comprising or, alternatively, consisting of at least one glycosaminoglycan, obtained from a plant starting material, selected from the group comprising or, alternatively, consisting of hyaluronic acid or a salt thereof (a hyaluronate anion salt) (in short, together or separate, HA), and/or chondroitin or a salt thereof such as a chondroitin sulfate or a salt thereof (in short, together or separate, CS), and the combinations thereof, of plant origin, having the characteristics as defined in the attached claims.

Furthermore, forming an object of the present invention is a use of the aforementioned mixture as an additive, excipient, or ingredient in the preparation of pharmaceutical products, medical devices, nutraceutical products, food for special medical purposes (FSMPs), food products or dietary supplements, said use having the characteristics as defined in the attached claims.

Forming another object of the present invention is a composition comprising (i) said mixture comprising or, alternatively consisting of hyaluronic acid or a salt thereof (a hyaluronate anion salt) (in short, together or separate, HA), and/or chondroitin or a salt thereof, such as chondroitin sulfate, (in short CS) of plant origin, and (ii) optionally technological additives and pharmaceutical or food grade excipients, having the characteristics as defined in the attached claims.

Forming another object of the present invention is a mixture and at least one technological additive or excipient, or a composition for use as medicament (first medical use), having the characteristics as defined in the attached claims.

Forming another object of the present invention is a mixture or a composition comprising said mixture for use in a method for the preventive or curative treatment of humans and animals having specific disorders or pathologies or diseases, selected from arthritis, osteoarthritis, arthrosis, joint pain, inflammation of the limbs and joints, gastroesophageal reflux (second medical use), said use having the characteristics as defined in the attached claims.

Furthermore, forming another object of the present invention is a process for preparing said mixture, or said composition comprising said mixture, comprising or, alternatively, consisting of hyaluronic acid or a salt thereof (a hyaluronate anion salt) (in short, together or separate, HA), and/or a chondroitin or a salt thereof, such as a chondroitin sulfate, (in short CS) of plant origin, having the characteristics as defined in the attached claims. Lastly, forming an object of the present invention is a use of a fungus as a plant starting material for preparing a hyaluronic acid or a salt thereof and/or a chondroitin or a salt thereof, such as chondroitin sulfate or a salt thereof, with a high degree of purity and a reduced content of contaminants and/or by-products, having the characteristics as defined in the attached claims.

Preferred embodiments of the present invention will now be described hereinafter, with the reference to the attached drawings, wherein:
- figures 1 to 4 show flow diagrams of the process, subject of the present invention, according to different embodiments (first embodiment, P1);
- figures 5 and 6 show flow diagrams of the process, subject of the present invention, according to the second embodiment (P2);
- figures 7 and 8 show flow diagrams of the process, subject of the present invention, according to the third embodiment (P3);

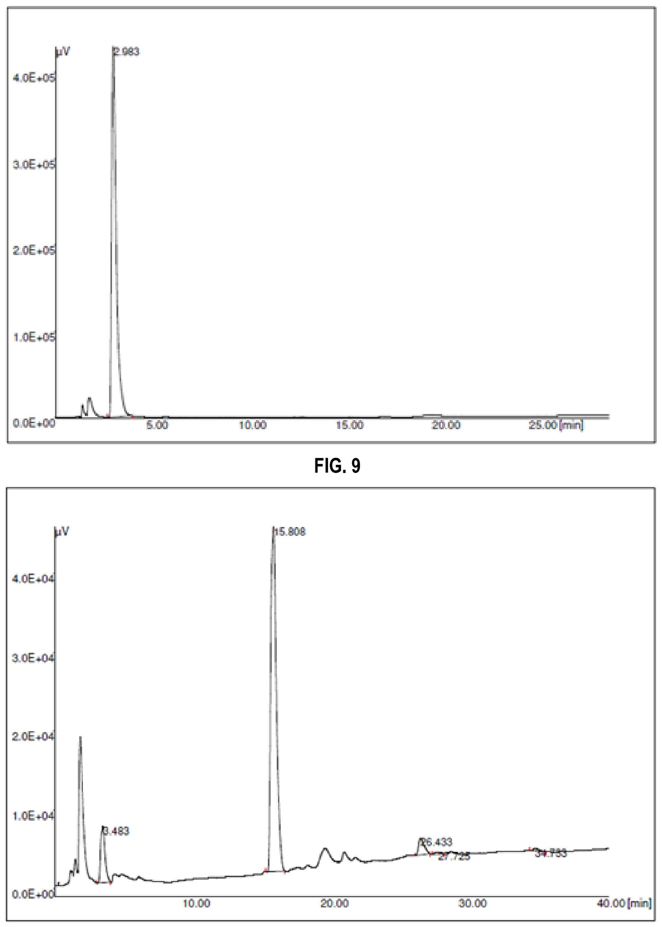

- figures 9 and 10 show two HPLC spectra for the determination of unsaturated disaccharides in a sample containing HA and in a sample containing CS, respectively.

It should be observed that, in the context of the present description, the expression "HS" is used to indicate hyaluronic acid or a salt thereof, or a hyaluronate, or combinations thereof. The expression "CS", on the other hand, is used to indicate a chondroitin, a chondroitin salt, preferably a chondroitin sulfate or a salt thereof, or mixtures thereof.

It should be observed that, in this description, the terms "plant starting material" and "starting material of plant origin" are synonyms, and are therefore used interchangeably.

### DETAILED DESCRIPTION OF THE INVENTION

Forming an object of the present invention is a mixture (m) comprising or, alternatively, consisting of at least one glycosaminoglycan obtained from a starting material of plant origin. Said material of plant origin is selected from the group comprising or, alternatively, consisting of one or more natural fungi.

Said glycosaminoglycan is selected from the group comprising or, alternatively, consisting of:
(a) a hyaluronic acid or a salt thereof, a hyaluronate anion (in short, HA);
(b) a chondroitin or a salt thereof, such as a chondroitin sulfate (in short, CS) or a salt thereof;
(c) a combination of (a) and (b).

The plant starting material is a fungus. The fungus is a fungus that grows and is found in nature, for example it can be found and collected in the woods, but it can also be cultivated in the greenhouse.

The fungus belongs to the *Dikarya* subkingdom, preferably it is of the *Basidiomycota* division.

Dikarya is a fungi subkingdom that includes the *Ascomycota* and *Basidiomycota* divisions. The Basidiomycetes (*Basidiomycota* R.T. Moore, 1980) is one of the largest *phyla* that form the kingdom of fungi.

According to an embodiment, the plant starting material is a fungus of the species *Tremella fuciformis* or said plant starting material comprises or, alternatively, consists of a fungus of the species *Tremella fuciformis.*

The *Tremella fuciformis* Berk. (1856) (also known as a snow fungus, or silver ear fungus) is a fungus originating in tropical and subtropical areas, where it thrives on dead hardwood logs, and it is also cultivated to cope with the extremely high demand - especially in Japan and China - in cooking and traditional medicine. *Tremella fuciformis* produces white gelatinous fruit-bearing bodies (basidiocarps), similar to fronds.

The use of *Tremella fuciformis* in the present invention is particularly advantageous given that, starting from this plant starting material, the extraction technology developed herein (first embodiment (P1), second embodiment (P2) and third embodiment (P3)), allow to produce both HA and CS completely of plant origin (and with low molecular weight). The HA and/or CS glycosaminoglycans contained in the mixture (m) and obtained from the process of the present invention have distinctive characteristics which make them particularly effective, above all by virtue of their reduced molecular weight, with respect to HA and/or CS obtainable from animal cartilage according to the prior art. A low molecular weight allows to have HA and/or CS glycosaminoglycans with improved transcutaneous penetration properties.

More precisely, hyaluronic acid or the salt thereof (hyaluronate) obtained by the process of the present invention (P1 and/or P2) has a weight average molecular weight comprised from 10 kDa to 600 kDa, preferably comprised from 100 kDa to 500 kDa, even more preferably comprised from 200 kDa to 400 kDa or from 100 kDa to 300 kDa, for example a weight average molecular weight of about 50 kDa, 150 kDa, or 250 kDa, or 300 kDa, or 350 kDa, or 450 kDa, or 550 kDa. Preferably, said HA contains a percentage by weight of chondroitin (preferably of non-sulfated chondroitin) comprised from 0.01% to 5%, preferably comprised from 0.1% to 3%, even more preferably comprised from 0.5% to 2%, for example 1% or 2%, with respect to the total weight of said HA extracted from fungus.

According to an advantageous aspect of the present invention, the HA having a weight average molecular weight falling within such ranges has a high transcutaneous penetration power due to the limited size of the molecule.

Chondroitin or the salt thereof, such as chondroitin or the salt thereof (CS) obtained by the process of the present invention (P1 and/or P3) has a weight average molecular weight comprised from 1 kDa to 50 kDa or from greater than 5 kDa to less than 50 kDa, preferably comprised from 3 kDa to 40 kDa, even more preferably comprised from 5 kDa to greater than 5 kDa to 25 kDa or from greater than 5 kDa to 10 kDa, for example a weight average molecular weight of about 4 kDa, or 6 kDa, or 8 kDa, or 10 kDa, or 12 kDa, or 14 kDa, or 16 kDa, or 18 kDa, or 22 kDa, or 24 kDa.

According to a further advantageous aspect of the present invention, the CS with a weight average molecular comprised in the ranges mentioned herein also proved to be effective in reducing bone damage from osteoarthritis of the knee and hip.

The CS contained in the mixture (m) of the present invention comprises a chondroitin sulfate having a weight average molecular weight comprised from 1 kDa (1,000.00 Da=1x10³ ) to 50 kDa, preferably comprised from 3 kDa to 40 kDa, even more preferably comprised from 5 kDa to 25 kDa, for example a weight average molecular weight of about 4 kDa, or 6 kDa, or 8 kDa, or 10 kDa, or 12 kDa, or 14 kDa, or 16 kDa, or 18 kDa, or 22 kDa, or 24 kDa. Preferably, said CS has a charge density of from 0.70 to 0.99 or from 0.70 to 1.50, preferably comprised from 0.75 to 0.98 or from 0.75 to 1.20, even more preferably comprised from 0.80 to 0.97, for example 0.85, 0.87, 0.90, 0.92, 0.94, or 0.96.

More preferably, said CS (obtained from the P1 and/or P3 process) has a percentage by weight of 6-chondroitin sulfate comprised from 50% to 99,5%, preferably comprised from 50% to 95%, more preferably comprised from 75% to 88%, even more preferably comprised from 78% to 86%, for example about 55%, 60%, 65%, 70%, 75%, 80%, 85%, 90%, 95% or 98%, with respect to the total weight of said CS (or with respect to the total of disaccharides contained in chondroitin sulfate; % determined, for example, by means of HPLC).

Besides 6-chondroitin sulfate, said CS preferably comprises non-sulfate chondroitin (non-sulfated chondroitin).

Preferably, the non-sulfated chondroitin has a weight percentage comprised from 0.1% to 25%, preferably from 0.5% to 20% or from 5% to 20%, more preferably comprised from 7% to 15%, even more preferably comprised from 8% to 13%, for example about 0.2%, 0.3%, 0.4%, 0.6%, 0.7%, 0.8%, 0.9%, 1%, 1.5%, 2%, 2.5%, 3%, 3.5%, 4%, 4.5%, 5.5%, 6%, 8%, 9%, 10%, 11% or 12%, with respect to the total weight of said CS (or with respect to the total of disaccharides contained in chondroitin sulfate; % determined, for example, by HPLC).

Besides 6-chondroitin sulfate and non-sulfated chondroitin, said CS preferably comprises 2.6-chondroitin disulfate. Preferably, the 2,6-chondroitin disulfate has a percentage by weight comprised from 0.1% to 10%, preferably comprised from 0.2% to 8%, even more preferably comprised from 0.3% to 5%, for example about 0.4%, 0.5%, 0.6%, 0.7%, 0.8%, 0.9%, 1%, 1.5%, 2%, 2.5%, 3%, 3.5%, 4% or 4.5%, with respect to the total weight of said CS (or with respect to the total of disaccharides contained in chondroitin sulfate; % determined, for example, by means of HPLC).

Besides 6-chondroitin sulfate, non-sulfated chondroitin and 2,6-chondroitin disulfate, said CS preferably comprise 4-chondroitin sulfate (or with respect to the total of disaccharides contained in chondroitin sulfate; % determined, for example, by means of HPLC).

Preferably, 4-chondroitin sulfate has a weight percentage comprised from 0.01% to 5%, preferably comprised from 0.05% to 3%, even more preferably comprised from 0.1% to 1.5%, for example about 0.02%, 0.03%, 0.04%, 0.1%, 0.15%, 0.2%, 0.25%, 0.3%, 0.35%, 0.4%, 0.45%, 0.5%, 0.6%, 0.7%, 0.8%, 0.9% or 1%, with respect to the total weight of said CS (or with respect to the total of disaccharides contained in chondroitin sulfate; % determined, for example, by means of HPLC).

Besides 6-chondroitin sulfate, non-sulfated chondroitin, 2,6-chondroitin disulfate and 4-chondroitin sulfate, said CS preferably comprises 4,6-chondroitin disulfate.

Preferably, 4,6-chondroitin disulfate has a weight percentage comprised from 0.01% to 5%, preferably comprised from 0.05% to 3%, even more preferably comprised from 0.1% to 1.5%, for example about 0.02%, 0.03%, 0.04%, 0.1%, 0.15%, 0.2%, 0.25%, 0.3%, 0.35%, 0.4%, 0.45%, 0.5%, 0.6%, 0.7%, 0.8%, 0.9% or 1%, with respect to the total weight of said CS (or with respect to the total of disaccharides contained in chondroitin sulfate; % determined, for example, by means of HPLC).

Besides 6-chondroitin sulfate, non-sulfated chondroitin and 2,6-chondroitin disulfate, 4-chondroitin sulfate and 4,6 chondroitin disulfate, said CS preferably comprise 2,4-chondroitin disulfate (or with respect to the total of disaccharides contained in chondroitin sulfate; % determined, for example, by means of HPLC).

Preferably, 2,4-chondroitin disulfate has a weight percentage comprised from 0.01% to 5%, preferably comprised from 0.05% to 3%, even more preferably comprised from 0.1% to 1.5%, for example about 0.02%, 0.03%, 0.04%, 0.1%, 0.15%, 0.2%, 0.25%, 0.3%, 0.35%, 0.4%, 0.45%, 0.5%, 0.6%, 0.7%, 0.8%, 0.9% or 1.0%, with respect to the total weight of said CS (or with respect to the total of disaccharides contained in chondroitin sulfate; % determined, for example, by means of HPLC).

Besides 6-chondroitin sulfate, non-sulfated chondroitin, 2,6-chondroitin disulfate, 4-chondroitin sulfate, 4,6-chondroitin disulfate and 2,4-chondroitin disulfate, said CS preferably comprises hyaluronic acid or hyaluronate (preferably non-sulfated). Preferably, said HA is present at a weight percentage comprised from 0.01% to 5%, preferably comprised from 0.05% to 3%, even more preferably comprised from 0.1% to 1.5%, for example 0.8% or 1.0%, with respect to the total weight of CS.

According to an embodiment, the CS contained in the mixture (m) and obtained by the process of the present invention (P1 and/or P3) comprises:
- a 6-chondroitin sulfate at a weight percentage comprised from 50% to 99.5%, preferably from 50% to 95±0.5%, more preferably comprised from 75% to 88%, even more preferably comprised from 78% to 86%;
- a non-sulfated chondroitin at a weight percentage comprised from 0.1% to 25%, preferably from 0.5% to 20%, more preferably comprised from 7% to 15%, even more preferably comprised from 8% and 13%;

- a 2,6-chondroitin disulfate at a weight percentage comprised from 0.1% to 10%, preferably comprised from 0.2% to 8%, even more preferably comprised from 0.3% to 5%; and, furthermore,
- a 4-chondroitin sulfate at a weight percentage comprised from 0.01% to 5%, preferably comprised from 0.05% to 3%, even more preferably comprised from 0.1% to 1.5%,
- a 4,6-chondroitin disulfate at a weight percentage comprised from 0.01% to 5%, preferably comprised from 0.05% to 3%, even more preferably comprised from 0.1% to 1.5%, and
- a 2,4-chondroitin disulfate at a weight percentage comprised from 0.01% to 5%, preferably comprised from 0.05% to 3%, even more preferably comprised from 0.1% to 1.5%.

By way of example, the CS contained in the mixture (m) and obtained by the process of the present invention has a composition CS.1, CS.2, CS.3, CS.4, CS.5 or a composition CS.6, according Table 1 below (the values being expressed as a weight percentage of each component with respect to the total weight of said CS.n with n=1-6).

**Table 1**

| **CS contained in the mixture (m)** | **CS.1** | **CS.2** | **CS.3** | **CS.4** | **CS.5** | **CS.6** |
|---|---|---|---|---|---|---|
| Non-sulfated chondroitin | 10.6 | 11.5 | 10.8 | 8.9 | 2.9 | 1.7 |
| 6-chondroitin sulfate | 85.3 | 79.7 | 84.7 | 87.9 | 92.6 | 95.7 |
| 4-chondroitin sulfate | 0.0 | 0.2 | 0.4 | 0.6 | 0.1 | 0.4 |
| 2,6-chondroitin disulfate | 4.1 | 0.5 | 2.4 | 1.9 | 3.4 | 0.5 |
| 4,6-chondroitin disulfate | 0.0 | 0.9 | 0.8 | 0.3 | 0.4 | 0.9 |
| 2,4-chondroitin disulfate | 0.0 | 1.0 | 0.9 | 0.4 | 0.6 | 0.8 |
| | | | | | | |
| Charge density | 0.94 | 0.85 | 0.95 | 0.92 | 0.94 | 0.96 |

Forming an object of the present invention is a composition comprising: (i) the aforementioned mixture (m) comprising or, alternatively, consisting of (a) HA having a weight average molecular weight comprised from 10 kDa to 600 kDa (preferably from 100 kDa to 500 kDa, even more preferably from 200 kDa to 400 kDa, Or from 100 kDa to 300 kDa, for example about 150 kDa, or 250 kDa, or 300 kDa, or 350 kDa, or 450 kDa, or 550 kDa), And/or (b) CS having a weight average molecular weight comprised from 1 kDa to 50 kDa or from greater than 5 kDa to less than 50 kDa (preferably comprised from 3 kDa to 40 kDa, even more preferably comprised from 5 or greater than 5 kDa to 25 kDa, for example about 4 kDa, or 6 kDa, or 8 kDa, or 10 kDa or 12 kDa, or 14 kDa, or 16 kDa, or 18 kDa, or 22 kDa, or 24 kDa), and (ii) optionally technological additives and pharmaceutical or food grade excipients. Such a composition may be a pharmaceutical composition, a medical device composition (EU) 2017/745, a nutraceutical function composition, a special food for medical purpose (SFMPs) composition, a dietary supplement composition, or a food product composition, or a novel food composition (EU) 2015/2283.

Such composition can be used as medicament, or as a composition for use in the preventive and/or curative treatment of arthritis, osteoarthritis, arthrosis, joint pain, inflammation of the limbs and joints, gastroesophageal reflux.

Forming an object of the present invention is a technology and a process for preparing hyaluronic acid or hyaluronate (HA) (process P1 and/or P2) and/or chondroitin sulfate or chondroitin or a salt thereof (CS) (process P1 and/or P3), said process comprising at least one step of extracting hyaluronic acid or hyaluronate and/or chondroitin sulfate or chondroitin from a starting material of plant origin, for example a starting material of plant origin comprising or, alternatively, consisting of at least one natural fungus belonging to the *Dikarya* subkingdom, preferably *Basidiomycota* division , more preferably *Tremella fuciformis* species.

The processes of the present invention (first embodiment (P1), second embodiment (P2) and third embodiment (P3)) do not include the steps of fermentation and/or digestion with bacteria, as for example the bacterial fermentations reported in patent documents WO 2012/152872 A1 and EP 2852437 B1 for the preparation of chondroitin or chondroitin sulfate.

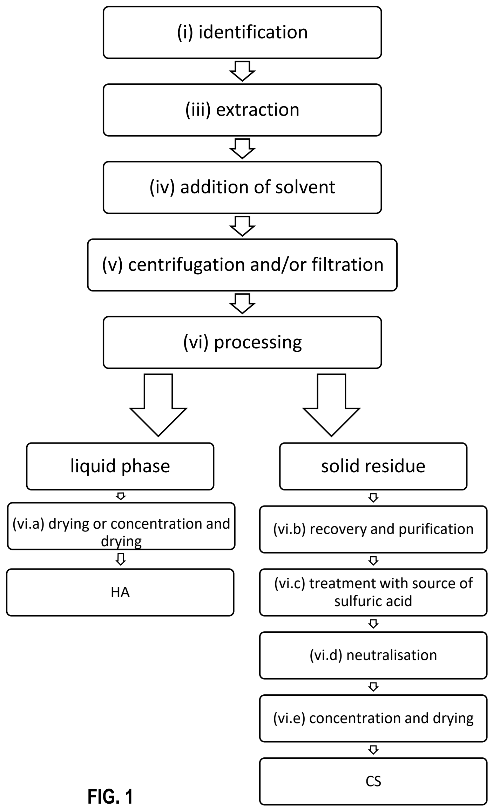

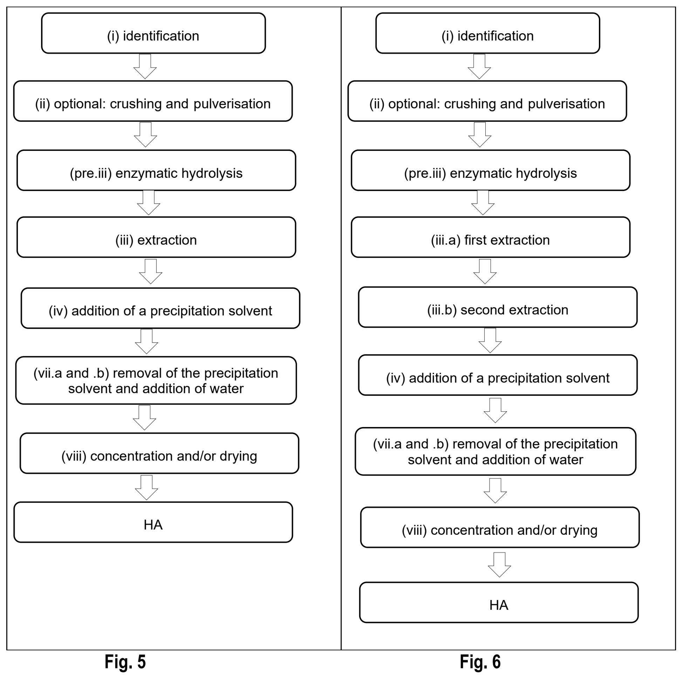

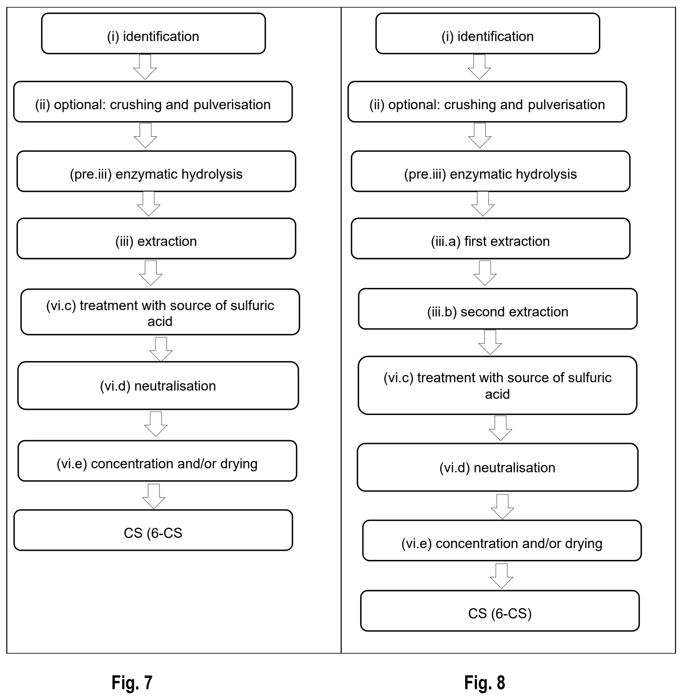

Various embodiments of this process are exemplified in the flow diagrams of figures 1 to 8.

According to a first embodiment (P1), the process subject of the present invention comprises the following steps:
(i) identifying one or more natural fungi as a plant starting material of a glycosaminoglycan; for example, one or more natural fungi in dry or dried form, preferably comprising or, alternatively, consisting of at least one fungus belonging to the *Dikarya* subkingdom, preferably *Basidiomycota* division, more preferably *Tremella fuciformis* species;
(ii) optionally, carrying out the crushing or pulverisation of the plant starting material;
(iii) extraction of said glycosaminoglycan (HA or CS) from the plant starting material obtained from step (i) or from step (ii) using an extraction solvent, preferably an aqueous solvent, even more preferably water (for example distilled or double distilled water) to obtain an aqueous extract of said glycosaminoglycan;
(iv) addition of a solvent, preferably ethanol, to the aqueous extract obtained from step (iii) to obtain a liquid product;
(v) carrying out a centrifugation and/or a filtration of the liquid product obtained from step (iv) to obtain a liquid phase and a solid residue;
(vi) carrying out a processing of the liquid phase obtained from the centrifugation and/or filtration of step (v) by means of the following step (vi.a), and/or processing the solid residue obtained from the centrifugation and/or filtration of step (v) by means of the following steps (vi.b), (vi.c), (vi.d) and (vi.e):
   (vi.a) drying, preferably concentration and drying, the liquid phase obtained from step (v) to obtain hyaluronic acid or the salt thereof having a weight average molecular weight comprised from 10 kDa to 600 kDa;
      and/or
   (vi.b) recovery and purification of the solid residue obtained from step (v) to obtain chondroitin or the salt thereof (CS) having a weight average molecular weight comprised from 1 kDa to 50 kDa;
   (vi.c) treating chondroitin or the salt thereof (CS) obtained from step (vi.b) with a source of sulfuric acid, preferably selected from the group comprising or, alternatively, consisting of sulfuric acid, a sulfur-trioxide pyridine complex, a sulfur-trioxide dimethyl-formamide complex and the mixtures thereof, to obtain an acidified product;
   (vi.d) neutralisation of the acidified product obtained from step (vi.c) using a basic agent to obtain a neutralised product;
   (vi.e) concentration and drying of the neutralised product obtained from step (vi.d) to obtain chondroitin sulfate or a salt thereof having a weight average molecular weight comprised from 1 kDa to 50 kDa.

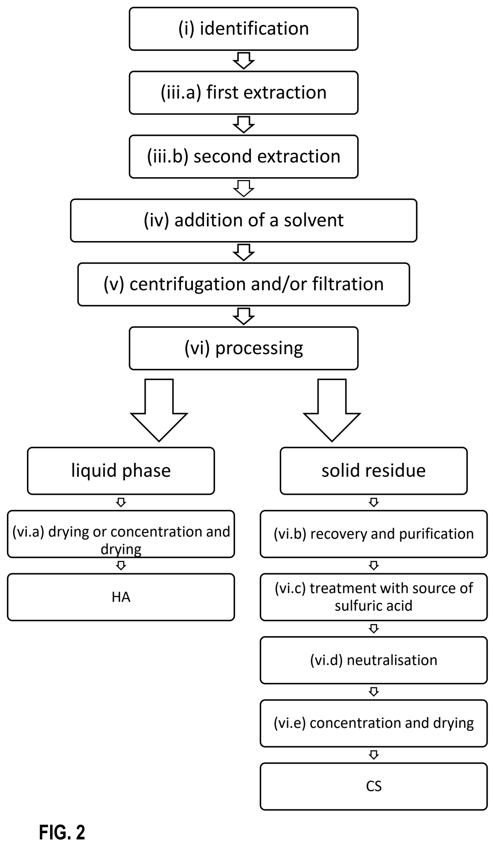

The plant starting material subjected to extraction in step (iii) could be intact (i.e. a single piece, for example a whole fungus), or it could be crushed (into pieces or flakes) or pulverised (into granules, powder or pellets) in step (ii). The flow diagrams of figure 1 and figure 2 show embodiments of the process subject of the present invention according to the first embodiment (P1) in which the natural fungus, or the plurality thereof, identified in step (i) it is extracted in step (iii) or steps (iii.a) and (iii.b) to obtain the aqueous extract.

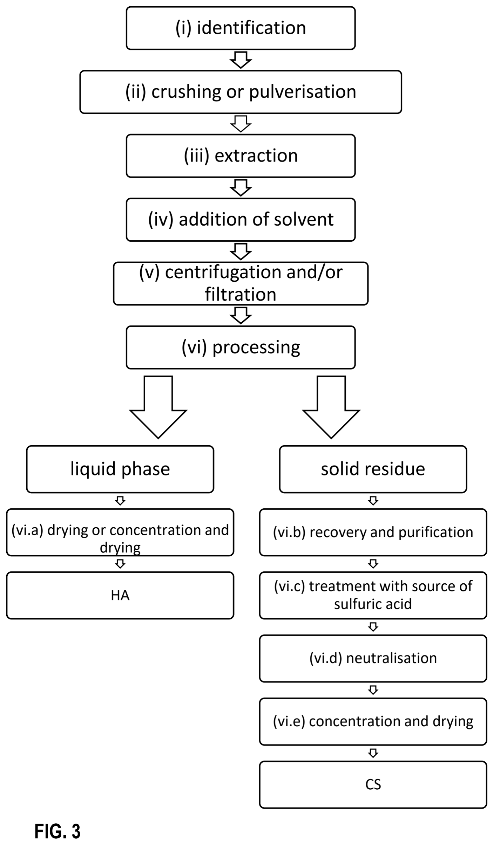

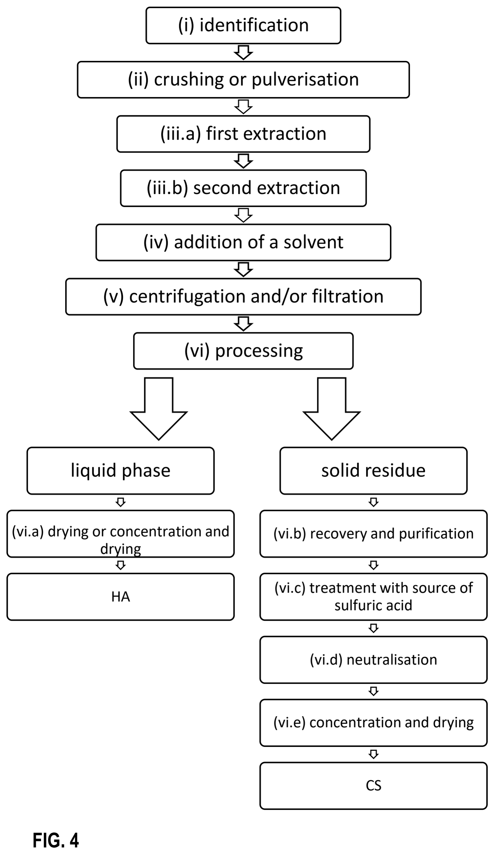

The flow diagrams of figure 3 and figure 4 show embodiments of the process subject of the present invention according to the first embodiment (P1) (process carried out according to techniques and apparatuses known to the man skilled in the art) in which the natural fungus, or the plurality thereof, identified in step (i), is crushed or pulverised in step (ii). Subsequently, the natural fungus, or the plurality thereof, crushed or pulverised in step (ii) is extracted in step (iii) or steps (iii.a) and (iii.b) to obtain the aqueous extract.

In the case where the plant starting material is crushed or pulverised (according to a technique and apparatus known to the man skilled in the art) in step (ii), an average distribution of the particle size of said plant starting material is preferably comprised from 500 µm and 2,500 µm, more preferably comprised from 800 µm to 1,800 µm, even more preferably comprised from 900 µm to 1,200 µm.

According to an embodiment, the plant starting material supplied in step (iii) (from step (i) or (ii)) is a plant material crushed into pieces or flakes, or pulverised either into granules or into pellets.

The plant starting material supplied in step (iii) (from step (i) or (ii)) is preferably dry or dried, that is to say it is a plant starting material containing an amount by weight of water comprised from about 2% to 20%, preferably comprised from 5% to 15%, even more preferably comprised from 8% to 10%, with respect to the total weight of the plant starting material.

In step (iii), the extraction solvent is selected from an aqueous solvent and water.

The aqueous solvent (or aqueous solution) is preferably a hydroalcoholic mixture in which the alcohol (for example ethanol) is present at a weight percentage comprised from 0.1% to 50%, more preferably comprised from 0.5% to 25%, even more preferably comprised from 1% to 15%, with respect to the total weight of the extraction solvent, for example at a percentage by weight of about 1.5%, 2%, 2.5%, 3%, 3.5%, 4%, 4.5%, 5% 7.5%, 10%, 20%.

The water is preferably distilled or double distilled water.

In step (iii), the plant starting material is loaded continuously or in batches into a container or extraction apparatus for example provided with mechanical stirring means, heating means, filtering means as well as temperature and pressure control means.

In step (iii), the plant starting material is then extracted in said extraction container or apparatus by means of the extraction solvent, so that hyaluronic acid or the salt thereof passes into solution in the liquid phase, inside the aqueous extract, and so that chondroitin remains in the solid residue.

The extraction of step (iii) is carried out using a [weight of the plant starting material] : [volume of the extraction solvent] ratio comprised from 1:1: to 1:90, preferably comprised from 1:10 to 1:90, more preferably comprised from 1:20 to 1:75, even more preferably comprised from 1:40 to 1:60, for example of 1:3, 1:5, 1:15, 1:25, 1:45, 1:50 or 1:55. The extraction of step (iii) is carried out within a period of time comprised from 1 minute to 12 hours, preferably from 10 minutes to 9 hours, even more preferably from 15 minutes to 4 hours, for example in about 30 minutes, 45 minutes, 60 minutes, 90 minutes, 120 minutes, 150 minutes, or 180 minutes.

Step (iii) is preferably carried out at atmospheric pressure (P=1 Atm. at 20-25°C), and at a temperature of the extraction solvent comprised from 10°C to 90°C, preferably comprised from 20°C to 60°C, even more preferably from 35°C to 55°C, for example of about 25°C, 30°C, 40°C, 44°C, 48°C, Or 50°C.

Preferably, a pH value of the extraction solvent in step (iii) is comprised from 3 to 10, preferably from 3.5 to 9, more preferably from 4 to 8, even more preferably comprised from 5 to 7, for example at a pH value of about 4.5; 5.5; 6; 6.5; 7.5; 8.5; or 9.5.

In the extraction of step (iii) a proteolytic enzyme is preferably used to degrade the surface pectins of the material of plant origin, and thus increase the yield of the process. Preferably, the proteolytic enzyme comprises or, alternatively, consists of bromelain or bromelain extract.

Bromelain is an enzyme extract of the fruit and/or stem of pineapple containing proteolytic enzymes and other substances in smaller quantities.

In order to increase the yield of the extraction, step (iii) is preferably carried out in two steps, as described below:
(iii.a) a first extraction from the plant starting material with a first volume of extraction solvent at a temperature comprised from 10°C to 90°C, preferably comprised from 20°C to 60°C, even more preferably comprised from 35°C to 55°C, for a period of time comprised from 1 minute or 30 minutes to 12 hours, preferably from 10 minutes to 9 hours, even more preferably from 15 minutes to 4 hours, to obtain a first aqueous extract; and
(iii.b) a second extraction from the plant starting material (or from a solid residue of said first extraction step (iii.a)) with a second volume of extraction solvent at a temperature comprised from 80°C to 120°C, preferably comprised from 90°C to 110°C, preferably comprised from 95°C to 105°C, even more preferably comprised from 98°C to 102°C, preferably under pressure or at reduced pressure, for a period of time comprised from 10 minutes to 6 hours or from 30 minutes to 8 hours, preferably from 20 minutes to 4 hours, even more preferably from 40 minutes to 2 hours, for example for a period of time of about 30 minutes, 60 minutes, or 90 minutes, to obtain a second aqueous extract. Preferably, in the first extraction (iii.a) a first volume of extraction solvent is used comprised from 25 to 75 times the weight of the plant starting material, preferably comprised from 35 and 65 times, even more preferably comprised from 45 to 55 times, for example about 30 times, or 50 times.

Preferably, in the second extraction (iii.b) a second volume of extraction solvent is used comprised from 10 and 150 times, preferably comprised from 75 to 125 times the weight of the plant starting material, more preferably comprised from 85 to 115 times, even more preferably comprised from 95 to 105 times, for example about 15, 20, 25, 30, 35, 40, 45, 50, 55, 60, 65, 70, 75, 80, 85, 90, 95 or 100 times.

According to said first embodiment of the HA and/or CS (P1) preparation process, the first aqueous extract obtained from step (iii.a) and the second aqueous extract obtained from step (iii.b) are then combined, and the solvent according to step (iv) is added thereto.

In step (iv), the solvent, preferably ethanol, is added to the aqueous extract obtained from step (iii), or to the first aqueous extract obtained from step (iii.a) and to the second aqueous extract obtained from step (iii.b) to obtain the liquid product.

In step (v), the liquid product obtained from step (iv) and the plant starting material present (for example as supernatant or as precipitate) in such product, are centrifuged and/or they are made to pass through a filtering means (first filtration) which retains the solid part (a solid residue), and which lets the liquid phase to pass through.

In step (vi), subsequent to step (v), the liquid phase obtained by centrifugation and/or filtration is processed through step (vi.a), and/or the solid residue is processed through steps (vi.b), (vi.c), (vi.d) and (vi.e).

In the preferred step (vi.a), the liquid phase obtained from step (v) is dried, optionally concentrated and dried, to obtain a hyaluronic acid or the salt thereof having a weight average molecular weight comprised from 10 kDa to 600 kDa.

The liquid phase to be dried, preferably to be concentrated and dried, in step (vi.a) must preferably be free of sediments, when allowed to cool to 20-25°C. Therefore, in the presence of sediments, the liquid phase obtained from step (v) is further centrifuged and/or filtered before step (vi.a).

The concentration of the centrifuged and/or filtered liquid phase is preferably carried out at a temperature comprised from 60°C to 90°C, more preferably comprised from 65°C to 85°C, even more preferably comprised from 70°C to 80°C, for example at 70°C, at 75°C or at 80°C. Besides the temperature, the duration of the concentration step also depends on the desired amount of substances dissolved in the liquid phase.

The concentration of the centrifuged and/or filtered liquid phase preferably provides for an increase in an amount of dissolved substances (including HA) in the liquid phase up to a range comprised from 1 g to 35 g per 100 ml of liquid phase, preferably from 5 g to 25 g, even more preferably comprised from 8 g to 18 g.

Preferably, the liquid phase obtained from the concentration step (vi.a) has a relative density (defined as the [density of the centrifuged and/or filtered liquid phase]: [density of the liquid phase at the end of the concentration]) ratio comprised from 1.01 to 1.20, preferably comprised from 1.02 to 1.15, even more preferably comprised from 1.05 to 1.08.

Preferably, the concentration of the liquid phase is carried out at reduced pressure (lower than 1 atm. att 25°C), more preferably at a pressure comprised from -1.5 mPa to -0.1 mPa, even more preferably comprised from -1.0 mPa to -0.5 mPa, for example to -0.8 mPa.

Preferably, the HA obtained from step (vi.a) has a purity (% by weight with respect to the total weight of HA) comprised from 90% to 100%, preferably comprised from 95% to 99.5%, even more preferably comprised from 97% to 99% (% determined, for example, by means of HPLC).

In the preferred steps (vi.b), (vi.c), (vi.d) and (vi.e), the solid residue obtained from centrifugation and/or filtration of step (v) is processed to obtain chondroitin sulfate or a salt having a weight average molecular weight comprised from 1 kDa to 50 kDa.

In the preferred step (vi.b), the solid residue obtained from centrifugation and/or by filtration of step (v) is recovered and purified to obtain chondroitin or salt thereof (CS) having a weight average molecular weight comprised from 1 kDa to 50 kDa.

In the preferred step (vi.c) (sulfonation (or sulfation) step, intended as a reaction step capable of inserting a sulfate group on the dimer of chondroitin) subsequent to step (vi.b), the solid residue is treated with the sulfuric acid source (preferably selected from the group comprising or, alternatively, consisting of sulfuric acid, a sulfur-trioxide pyridine complex, a sulfur-trioxide dimethyl-formamide complex and mixtures thereof), to obtain the acidified product (in which said sulfonation step is carried out according to a technique and apparatus known to the man skilled in the art).

The amount of the sulfuric acid source used in step (vi.c) is such to obtain a weight percentage of 6-chondroitin sulfate comprised from 51% to 99%, or about 95±0.5% (preferably comprised from 78% to 85%, or 86%) with respect to the total content of disaccharides of CS in the solid residue of step (vi.b).

Preferably, in step (vi.c) there are used from 1 ml to 50 ml of the sulfur-trioxide dimethyl-formamide complex (SO₃ DMF), preferably from 2 ml to 40 ml, even more preferably from 4 ml to 30 ml, for every 100 g of chondroitin or salt thereof (CS) obtained from step (vi.b), for example 5 ml, 10 ml, 15 ml, 18 ml, 22 ml or 25 ml of the sulfur-trioxide dimethyl-formamide complex every 100 g of the chondroitin or of the salt thereof (CS) obtained from step (vi.b). More preferably, the sulfur-trioxide dimethylformamide complex is added to the chondroitin or to the salt thereof (CS) obtained from step (vi.b) in several steps, for example by adding from 2 ml to 8 ml, then adding from 8 ml to 12 ml, and lastly adding further from 8 ml to 12 ml of the sulfur-trioxide dimethyl-formamide complex.

The treatment of step (vi.c) is carried out for a period of time comprised from 1 minute to 4 hours, preferably comprised from 10 minutes to 2 hours, even more preferably comprised from 20 minutes to 1 hour, at a temperature comprised from 20°C to 80°C, preferably comprised from 30°C to 70°C, even more preferably comprised from 40°C to 60°C. In step (vi.d), the product obtained from step (vi.c) is neutralised with a basic agent.

Therefore, in step (vi.d) the source of sulfuric acid still free in the acidified product of step (vi.c) (i.e. the source of sulfuric acid not bound to chondroitin as chondroitin sulfate in the acidified product) is eliminated by neutralising with the basic agent to obtain the neutralised product.

In the present description, the expression "neutralised" or "neutralisation" is used to indicate a pH value of 6 to 8, preferably comprised from 6.4 to 7.6, even more preferably comprised from 6.6 to 7.4, for example at a pH value of 7.0±0.2.

The basic agent used in step (vi.d) is preferably an inorganic basic agent.

The basic agent is preferably selected from the group comprising or, alternatively, consisting of: ammonia, sodium hydroxide, potassium hydroxide and mixtures thereof.

Preferably, the sodium hydroxide usable in step (vi.d) is at a concentration of 1 M, 2 M or 4 M.

In the preferred strep (vi.e), subsequent to step (vi.d), the neutralised product obtained from step (vi.d) is concentrated and dried to obtain chondroitin sulfate having a weight average molecular weight comprised from 1 kDa to 50 kDa

The concentration of step (vi.e) provides for a relative density (defined as the [density of the neutralized product obtained from step (vi.d)]: [density of the concentrated product obtained from step (vi.e)]) ratio comprised from 1.0 to 1.30, preferably comprised from 1.01 to 1.20, even more preferably comprised from 1.05 to 1.15.

Preferably, the concentration of step (vi.e) is carried out by means of dialysis and/or through vacuum concentration. More preferably, dialysis is carried out by means of a dialysis bag, so as to remove small impurities which may be present.

The concentration step (vi.e) is preferably terminated when the solid content in the concentrated product obtained from step (vi.e) is comprised from 10 g to 60 g per 100 ml, preferably comprised from 20 g to 50 g per 100 ml, even more preferably comprised from 35 g to 45 g per 100 ml, for example 40 g/100 ml.

The drying of step (vi.e) is carried out subsequently to the concentration of step (vi.e), preferably by means of a vacuum oven.

Forming an object of the present invention is a use of a plant starting material, preferably a fungus, more preferably of the *Dikarya* subkingdom, even more preferably of the *Basidiomycota* division, further preferably of the *Tremella fuciformis* species, for preparing a hyaluronic acid or a salt thereof (HA), a hyaluronate anion salt, and/or a chondroitin or a salt thereof, such as a chondroitin sulfate or a salt thereof (CS).

The embodiments of the mixture, of the use of said mixture, of the composition comprising said mixture as additive, or excipient, or ingredient (or non-active ingredient), of the use of the composition as medicament, of the use of the composition in the treatment of specific disorders or diseases or pathologies, of the process for preparing said mixture or of said composition comprising said mixture, of the use of said composition for use as additive, or excipient, or ingredient, and of the aforementioned plant starting material could be subjected - by a man skilled in the art - to substitutions or modifications regarding the described characteristics according to the contingencies. These embodiments are also to be considered included in the scope of protection formalised in the following claims. Furthermore, it should be observed that any embodiment may be implemented independently from the other embodiments described.

Embodiments present invention (FRn) are outlined below:
FR1. A mixture (M) comprising or, alternatively consisting of a glycosaminoglycan obtained from a plant starting material; said glycosaminoglycan being selected from the group comprising or, alternatively consisting of:
   (a) hyaluronic acid or a salt thereof, a hyaluronate anion, (HA) having a weight average molecular weight comprised from 10 kDa to 600 kDa;
   (b) chondroitin or a salt thereof (CS) such as chondroitin sulfate, having a weight average molecular weight comprised from 1 kDa to 50 kDa;
   (c) a combination of (a) and (b).
FR2. The mixture (M) according to the preceding FR, wherein:
   (a) the hyaluronic acid or the salt thereof (HA) has a weight average molecular weight comprised from 100 kDa to 500 kDa, preferably comprised from 200 kDa to 400 kDa; and/or
   (b) the chondroitin or the salt thereof (CS) has a weight average molecular weight comprised from 1 kDa to 50 kDa, preferably comprised from 3 kDa to 40 kDa, even more preferably comprised from 5 kDa to 25 kDa.
FR3. The mixture (M) according to any one of the preceding FRs, wherein said material of plant starting material is a fungus, preferably of the *Dikarya* subkingdom, even more preferably of the *Basidiomycota* division.
FR4. The mixture (M) according to the preceding FR, wherein the fungus is of the *Tremella fuciformis* species. FR5. Use of the mixture (M) according to anyone of the preceding FRs, as additive, excipient, or ingredient in the preparation of pharmaceutical products, medical devices, nutraceutical products, food for special medical purposes (SFMPs), dietary supplements or food products.
FR6. A composition comprising: (i) the mixture according to any one of FR1-4, and (ii) technological additives or pharmaceutical or food grade excipients.
FR7. The composition according to the preceding FR, for use as medicament.
FR8. The composition according to FR6, for use in the preventive or curative treatment of human beings or animals, having specific disorders or diseases, selected from arthritis, osteoarthritis, arthrosis, joint pain, inflammations of the limbs and joints, or gastroesophageal reflux, and/or for use as an additive, or excipient, or ingredient in the preparation of pharmaceutical products, products for medical devices, nutraceutical products, food for special medical purpose (FSMPs), food products or food supplements.
FR9. A process for the preparation of hyaluronic acid or hyaluronate (HA), and/or chondroitin sulfate or chondroitin (CS), said process comprising at least one step for extracting hyaluronic acid or hyaluronate, and/or chondroitin sulfate or chondroitin, from a starting material of plant origin.
FR10. The process according to the preceding FR, comprising the following steps:
   (i) identifying one or more natural fungi as the plant starting material of a glycosaminoglycan;
   (ii) optionally crushing or pulverising the plant starting material;
   (iii) extraction of said glycosaminoglycan from the plant starting material obtained from step (i) or from step (ii) with an extraction solvent, preferably an aqueous solvent, even more preferably water to obtain an aqueous extract of said glycosaminoglycan;
   (iv) addition of a solvent, preferably ethanol, to the aqueous extract obtained from step (iii) to obtain a liquid product;
   (v) centrifugation and/or filtration of the liquid product obtained from step (iv) to obtain a liquid phase and a solid residue;
   (vi) processing the liquid phase obtained from the centrifugation and/or filtration of step (v) by means of the following step (vi.a), and/or processing the solid residue obtained from the centrifugation and/or filtration of step (v) by means of the following steps (vi.b), (vi.c), (vi.d) and (vi.e):
      (vi.a) drying, preferably concentration and drying, the liquid phase obtained from step (v) to obtain hyaluronic acid or the salt thereof having a weight average molecular weight comprised from 10 kDa to 600 kDa; and/or
      (vi.b) recovery and purification of the solid residue obtained from step (v) to obtain chondroitin or the salt thereof (CS) having a weight average molecular weight comprised from 1 kDa to 50 kDa;
      (vi.c) treating chondroitin or the salt thereof (CS) obtained from step (vi.b) with a source of sulfuric acid, preferably selected from the group comprising or, alternatively, consisting of sulfuric acid, a sulfur-trioxide pyridine complex, a sulfur-trioxide dimethyl-formamide complex and the mixtures thereof, to obtain an acidified product;
      (vi.d) neutralisation of the acidified product obtained from step (vi.c) using a basic agent to obtain a neutralised product;
      (vi.e) concentration and drying of the neutralised product obtained from step (vi.d) to obtain chondroitin sulfate having a weight average molecular weight comprised from 1 kDa to 50 kDa.
FR11. Use of a plant starting material, preferably a fungus, more preferably of the *Dikarya* subkingdom, even more preferably of the *Basidiomycota* division, further preferably of the *Tremella fuciformis* species, for preparing a hyaluronic acid or a salt thereof, a hyaluronate anion salt, and/or a chondroitin or a salt thereof, such as a chondroitin sulfate.

A second embodiment of the process of the present invention (in short, P2) relates to a process for preparing hyaluronic acid or a salt thereof (HA), the process comprises or, alternatively, consists of the following steps:
(i) identifying one or more natural fungi as a plant starting material of hyaluronic acid; for example, one or more natural fungi in dry or dried form, preferably comprising or, alternatively, consisting of at least one fungus belonging to the *Dikarya* subkingdom, preferably *Basidiomycota* division, more preferably *Tremella fuciformis* species;
(ii) optionally, carrying out the crushing or pulverisation of the plant starting material (average particle size distribution preferably comprised from 500 µm to 1,800 µm, preferably from 700 µm to 1,000 µm, for example about 20 mesh = 841 µm);
   (pre-iii) carrying out an enzymatic hydrolysis of the plant starting material obtained from step (i) or step (ii) in an aqueous hydrolysis solvent, preferably water, at a temperature comprised from 10°C to 90°C wherein an enzyme, preferably a proteolytic enzyme, is added to the plant starting material dispersed in a volume of hydrolysis solvent to obtain a mixture of step (pre-iii);
(iii) extracting said mixture of step (pre-iii) with an aqueous extraction solvent, preferably water, at a temperature comprised from 91°C to 110°C, preferably comprised from 95°C to 110°C, even more preferably from 98°C to 105°C (for example about 100°C or boiling temperature of the extraction solvent), to obtain an aqueous extract;
(iv) adding a precipitation solvent (precipitation by adding a solvent), preferably an alcoholic solvent, more preferably ethanol, to the aqueous extract obtained from step (iii) to obtain a liquid product of step (iv); preferably, precipitating with ethanol by slowly adding 95% ethanol to said aqueous extract obtained from step (iii) at a volume/volume ratio comprised from 2 to 4, preferably 3, and maintaining under stirring within a period of time comprised from 8 hours to 16 hours, for example about 12 hours;
(vii) processing said liquid product of step (iv) by applying step (vii.a) and, optionally, step (vii. b):
   (vii. a) removing said precipitation solvent (for example by distillation or heating to a pressure value lower than the room temperature), preferably ethanol, to obtain a liquid product of step (vii. a);
   (vii.b) adding water to said liquid product of step (vii.a) (for example to dissolve solid products) to obtain a liquid product of step (vii.b);
   (viii) drying said liquid product of step (vii.b), preferably concentrating and/or drying (for example removing water and the possible residual precipitation solvent (e.g. ethanol) by concentration and/or drying and/or freeze-drying, preferably freeze-drying) to obtain a product PR1 comprising or, alternatively, consisting of hyaluronic acid or a salt thereof having a weight average molecular weight comprised from 10 kDa to 600 kDa, preferably from 50 kDa to 350 kDa, more preferably from 100 kDa to 300 kDa, and a purity comprised from 85% to about 100% with respect to the total weight of said product PR1, preferably comprised from 95% to 99.5%, even more preferably comprised from 97% to 99% (% determined, for example, by means of HPLC).

A third embodiment of the process of the present invention (in short, P3) relates to a process for preparing chondroitin sulfate, preferably 6-chondroitin sulfate, the process comprising or, alternatively consisting of the following steps:
(i) identifying one or more natural fungi as a plant starting material of hyaluronic acid; for example, one or more natural fungi in dry or dried form, preferably comprising or, alternatively, consisting of at least one fungus belonging to the *Dikarya* subkingdom, preferably *Basidiomycota* division, more preferably *Tremella fuciformis* species;
(ii) optionally, carrying out the crushing or pulverisation of the plant starting material (average particle size distribution preferably comprised from 500 µm to 1,800 µm, preferably from 700 µm to 1,000 µm, for example about 20 mesh = 841 µm);
   (pre-iii) carrying out an enzymatic hydrolysis of the plant starting material obtained from step (i) or step (ii) in an aqueous hydrolysis solvent, preferably water, at a temperature comprised from 10°C to 90°C wherein an enzyme, preferably a proteolytic enzyme, is added to the plant starting material dispersed in a volume of hydrolysis solvent to obtain a mixture of step (pre-iii);
(iii) extracting said mixture of step (pre-iii) with an aqueous extraction solvent, preferably water, at a temperature comprised from 91°C to 110°C, preferably comprised from 95°C to 110°C, even more preferably from 98°C to 105°C (for example about 100°C or boiling temperature of the extraction solvent), to obtain an aqueous extract;
   (vi.c.) treating with a source of sulfuric acid (sulfonation step) said aqueous extract to obtain a liquid product of step (vi.c), wherein said source of sulfuric acid is preferably selected from the group comprising or, alternatively, consisting of sulfuric acid, a sulfur-trioxide pyridine complex, a sulfur-trioxide dimethyl-formamide complex and mixtures thereof; more preferably the sulfur trioxide dimethylformamide complex (SO₃-DMS);
   (vi.d) neutralising said liquid product of step (vi.c) by adding a base to obtain a neutralised product, wherein said base is preferably an inorganic base, more preferably NaOH o KOH o Ca(OH)₂ or Mg(OH)₂, up to neutral pH;
   (vi.e) concentrating and drying said neutralised product obtained from step (vi.d) to obtain a product PR2 comprising or, alternatively, consisting of chondroitin sulfate or a salt thereof (in short, CS), having a weight average molecular weight comprised from 1 kDa to 45 kDa or 50 kDa (or from greater than 5 kDa to less than 50 kDa), preferably comprised from 3 kDa to 40 kDa, even more preferably comprised from 5 kDa or greater than 5 kDa to 25 kDa or from 5 kDa or greater than 5 kDa to 10 kDa, for example about 5 kDa, or 6 kDa, or 7 kDa, or 8 kDa, or 9 kDa, or 10 kDa.

The term "chondroitin sulfate or a salt thereof (in short, CS)" obtained from said third embodiment of the process of the present invention (P3) is used to indicate the combination of non-sulfated chondroitin and mono-, di- and/or tri-sulfated chondroitin in the various possible positions, preferably predominantly 6-chondroitin sulfate.

Alternatively, the term "chondroitin sulfate or a salt thereof (in short, CS)" obtained from said third embodiment of the process of the present invention (P3) is used to indicate the group of mono-, di- and/or tri-sulfated chondroitin in the various possible positions, preferably predominantly 6-chondroitin sulfate.

Preferably, said chondroitin sulfate (CS) obtained from said third embodiment of the process of the present invention (P3) has a weight average molecular weight in the ranges described (preferably from 5 kDa or greater than 5 kDa to 10 kDa, for example about 6 kDa, or 7 kDa, or 8 kDa, or 9 kDa), comprises 6-chondroitin sulfate at a weight percentage comprised from 51% to about 95±0.5%, preferably from 75% to 90%, even more preferably from 78% to 86%, with respect to the total weight of said chondroitin sulfate (CS) (or with respect to the total of disaccharides contained in chondroitin sulfate), and it has a purity comprised from 80% to 99.99% (for example, 94.5%, 94.6%, 94.7%, 94.8% or 94.9%) with respect to the total weight of the product PR2 obtained from step (vi.e), preferably comprised from 85% to 98% (for example, 86%, 87%, 88%, 89% or 89,5%), even more preferably comprised from 90% and 94.9%, for example 91%, 92%, 93%, 94%, or 94.5% (% determined, for example, by means of HPLC).

Even more preferably, said chondroitin sulfate (CS) obtained from said third embodiment of the process of the present invention (P3) has a weight average molecular weight in the ranges described (preferably from 5 kDa or greater than 5 kDa to 10 kDa, for example about 6 kDa, or 7 kDa, Or 8 kDa, or 9 kDa), comprises 6-chondroitin sulfate at a weight percentage comprised from 51% to about 95±0.5%, (preferably from 75% to 90%, more preferably from 78% to 86%), and 4-chondroitin sulfate at a weight percentage comprised from 0.01% to about 5%, (preferably from 0.05% to 3%, more preferably from 0.1% to 1.5%) with respect to the total weight of said chondroitin sulfate (CS) (or with respect to the total of disaccharides contained in chondroitin sulfate), and it has a purity comprised from 80% to 99.99% with respect to the total weight of the product PR2 obtained from step (vi.e), preferably comprised from 85% to 98% (for example, 86%, 87%, 88% or 89%), more preferably comprised from 90% to 95%, for example 91%, 92%, 93%, 94% or 94.5% (% determined, for example, by means of HPLC).

The weight average molecular weight of HA and/or CS can be calculated according to methods and instruments common and known to the man skilled in the art, for example, by means of high performance size exclusion chromatography (HPSEC); preferably, the weight average molecular weight of HA and/or CS can be determined by means of HPSEC provided with integrated specialised gel permeation chromatography (GPC) software.

In the step (pre-iii) of enzymatic hydrolysis, present in the second and in the third embodiments of the process of the present invention (P2 and P3), the enzyme (for example pectinase and/or cellulase and/or proteinase) is added to the plant starting material as described in the present invention, in a volume of hydrolysis solvent (for example an aqueous solvent or water comprised from 25 to 100 times the weight of the plant starting material, preferably comprised from 35 to 75 times, more preferably comprised from 45 to 55 times (for example 50 times), and heated to a temperature comprised from 10°C to 90°C, preferably from 20°C to 65°C, more preferably from 45°C to 55°C (e.g. about 50°C), for a period of time comprised from 0.5 hours to 12 hours, preferably from 1 hour to 8 hours, even more preferably from 2 hours to 6 hours (for example about 4 hours), to obtain said mixture of step (pre-iii) to be subjected to the extraction step (iii) or to the first extraction step (iii.a) (as described hereinafter).

Furthermore, advantageously, said enzymatic hydrolysis step (pre-iii) is carried out at a pH value of the enzymatic hydrolysis solution comprised from 2 to 9, preferably from 3 to 5 or from 5 to 8, more preferably from 3 to 4 (for example 3.5) or from 6 to 7; and/or in said enzymatic hydrolysis step (pre-iii) an amount of enzyme (aqueous solution of the enzyme at 1%-20%, or 2%-10%, or 3%-6% weight/weight or weight/volume) is used at a volume percentage comprised from 0.001% to 1%, preferably from 0.005% to 0.1%, more preferably from 0.008% to 0.05% (for example 0.01), with respect to the mass of the plant starting material to be extracted (volume/mass) or, alternatively, with respect to the volume of the solution of the plant starting material to be extracted in the hydrolysis solvent (volume/volume).

According to a preferred example, the enzymatic hydrolysis step (pre-iii) is carried out under the following conditions (approximately): volume 0.01%; temperature 50°C; actual pH 3.5; duration 4 hours.

Said enzyme used in step (pre-iii) of the second and/or third embodiment (P2 and/or P3) may be a pectinase and/or cellulase and/or proteinase.

Examples of enzymes usable in the process of the present invention are:
- commercial product Pectinex ^{®} Ultra Tropical, composition: enzyme: pectin lyase or pectinase, preservatives: potassium sorbate, stabilisers: sucrose, glycerol, sorbitol, sodium chloride, potassium chloride; compound activity: pectin lyase or pectinase (PECTU) = 5,000 PECTU/g; approximate density 1.18 (g/ml); pectin lyase is an enzyme which catalyses the elimination cleavage of (1,4)-alpha-D-galacturonate methyl ester which gives oligosaccharides with 4-deoxy-6- O-methyl-alpha-D-galact-4-enurosyl groups at the non-reducing ends thereof; other activities: cellulase, polygalacturonase, beta-glucanase (endo-1,3(4)-).
- commercial product Pectinex^{®} Ultra SP-L, composition % w/w: 45% glycerol (CAS N° 56-81-5), 45% water (CAS N° 7732-18-5), 5% polygalacturonase (CAS N° 9032-75-1; defined as enzyme concentration (on dry weight basis)), 5% potassium chloride (CAS N° 7447-40-7); compound activity: polygalacturonase (PGNU) = 3300 PGNU/g; approximate density 1,17 (g/ml); polygalacturonase is an enzyme that hydrolyses (1,4)-alpha-D-galactosiduronic bonds in pectate and other galacturonans.
- commercial product Viscozyme ^{®}, composition % w/w: 56.8% water (CAS N° 7732-18-5), 9% beta-glucanase (endo-1,3(4)-) (CAS N° 62213-14-3; defined as enzyme concentration (on dry weight basis)), 24% sucrose (CAS N° 57-50-1), 10% sodium chloride (CAS N° 7647-14-5), 0.20% potassium sorbate (CAS N° 24634-61-5); compound activity: beta-glucanase (endo-1,3(4)-) (FGB) = 100 FBG/g; approximate density 1.21 (g/ml); endo-beta-glucanase is an enzyme which hydrolyses (1,3)- or (1,4)- bonds in beta-D-glucans, other activities: xylanase, cellulase, hemicellulase.

In the second and third embodiments of the process of the present invention (P2 and/or P3), the extraction step (iii) preferably comprises or, alternatively, consists of the first extraction steps (iii.a) comprising or, alternatively, consisting of extracting with a first volume of extraction solvent at a temperature comprised from 91°C to 110°C (for example about 100°C) or a boiling temperature of the extraction solvent for a period of time comprised from 0.5 hours to 12 hours, preferably from 1 hour to 9 hours, more preferably from 1 hour to 4 hours (for example about 2 hours or 3 hours), to obtain a first aqueous extract; followed by the second extraction step (iii.b) comprising or, alternatively, consisting of extracting with a second volume of extraction solvent at a temperature comprised from 90°C to 110°C (for example about 100°C) or boiling temperature of the extraction solvent for a period of time comprised from 0.5 hours to 8 hours, preferably from 0.5 hours to 4 hours, more preferably from 1 hour to 3 hours (for example 1.5 hours, or 2 hours, or 2.5 hours), to obtain a second aqueous extract; said first extract and said second extract are combined to obtain a final aqueous extract; and, optionally, a step (iii.c) of concentrating said final aqueous extract to obtain a concentrated aqueous extract follows.

In the second and third embodiment of the process of the present invention (P2 and P3), preferably, in the first extraction (iii.a) a first volume of extraction solvent is used comprised from 25 to 100 times the weight of the plant starting material, preferably comprised from 35 and 65 times, even more preferably comprised from 45 to 55 times, for example 50 times. For example, said first extraction volume comprised from 25 to 100 times the weight of the plant starting material is added to the volume of enzymatic hydrolysis solvent, reaching a total volume of solvent comprised from 50 to 200 times the weight of the plant material (for example about 100 times).

In the second and third embodiment of the process of the present invention (P2 and P3), preferably, in the second extraction (iii.b) a second volume of extraction solvent is used comprised from 25 to 75 times the weight of the plant starting material, preferably comprised from 35 and 65 times, more preferably comprised from 45 to 55 times, (for example about 50 times).

In the second and third embodiments of the process of the present invention (P2 and P3), after the first extraction step (iii.a) a filtration of step (iii.a) is carried out: the filtrate corresponds to the first aqueous extract obtained from step (iii.a) and the residue is subjected to the second extraction step (iii.b). After the second extraction step (iii.b), a filtration step (iii.b) is carried out and the filtrate corresponds to the second aqueous extract obtained from step (iii.b). For example, said filtrations of step (iii.a) and (iii.b) (or filtrations of the extraction step (iii)) are carried out with filters from 140 to 270 mesh, preferably 200 mesh. Optionally, in said second and third embodiments (P2 and P3), the first aqueous extract obtained from step (iii.a) and the second aqueous extract obtained from step (iii.b) are combined and subjected to the vacuum concentration step (iii.c), for example at a temperature comprised from 60°C to 90°C, preferably from 70°C to 80°C, more preferably at about 75°C, up to a relative density comprised from about 0.8 to 1.5, preferably from 1.00 to 1.20 (for example about 1.05-1.08).

In the third embodiment of the process of the present invention (P3), preferably, in step (vi.c) (sulfonation step) there are used from 1 ml to 50 ml of the sulfur-trioxide dimethyl-formamide complex (SO₃ DMF), preferably from 2 ml to 40 ml, even more preferably from 4 ml to 30 ml, for every 100 g of chondroitin or a salt thereof (CS) obtained from step (vi.b), for example about 5 ml, 10 ml, 15 ml, 18 ml, 22 ml or 25 ml of the sulfur-trioxide dimethyl-formamide complex every 100 g of the chondroitin or of the salt thereof (CS) obtained from step (vi.b). More preferably, the sulfur-trioxide dimethylformamide complex is added to the chondroitin or to the salt thereof (CS) obtained from step (vi.b) in several steps, for example by adding from 2 ml to 8 ml, then adding from 8 ml to 12 ml, and lastly adding further from 8 ml to 12 ml of the sulfur-trioxide dimethyl-formamide complex. The treatment of step (vi.c) is carried out for a period of time comprised from 1 minute to 4 hours, preferably comprised from 10 minutes to 2 hours, even more preferably comprised from 20 minutes to 60 minutes (for example about 30 minutes), at a temperature comprised from 20°C to 80°C, preferably comprised from 30°C to 70°C, even more preferably comprised from 40°C to 60°C (for example about 50°C). According to a preferred example, said sulfonation step (vi.c) is carried out by adding to about 100 ml of aqueous extract deriving from step (iii) or (iii.b) or (iii.c) (solid content 10-15g/100 ml and relative density 1.05-1.08) SO₃-DMF (5 ml, 15 ml, 25 ml) at a temperature comprised from 40°C to 60°C (for example at 50°C) and for a period of time comprised from 20 minutes to 60 minutes (for example 30 minutes).

In the third embodiment of the process of the present invention (P3), preferably, the basic agent used in step (vi.d) is preferably an inorganic basic agent selected from the group comprising or, alternatively, consisting of: ammonia, sodium hydroxide, potassium hydroxide and mixtures thereof, preferably sodium hydroxide (for example, at a concentration of 1 M, 2 M or 4 M).

In the third embodiment of the process of the present invention (P3), preferably, said step (vi.e) comprises a membrane filtration by means of dialysis, for example by means of a 1,000 Da dialysis bag for a period of time comprised from 18 hours to 36 hours, preferably 24 hours, until a relative density comprised from 1.3 to 1.5, preferably about 1.1, is reached, followed by drying, for example in a vacuum oven.

According to an aspect of the invention, in said first and third embodiment of the process of the present invention (P1 and P3), the chondroitin sulfate or a salt thereof (CS) of the present invention has a weight average molecular weight comprised from greater than 5 kDa to less than 50 kDa, preferably from greater than 5 kDa to 25 kDa, and it comprises:
- a 6-chondroitin sulfate at a weight percentage comprised from 50% to 95±0.5%, preferably from 75% to 90%;
- a non-sulfated chondroitin at a weight percentage comprised from 5% to 20%, preferably from 7% to 15%;
- a 2,6-chondroitin disulfate at a weight percentage comprised from 0.1% to 10%, preferably from 0.2% to 8%; and
- a 4-chondroitin sulfate at a weight percentage comprised from 0.01% to 5%, preferably from 0.05% to 3%, all the percentages being expressed with respect to the total of disaccharides contained in chondroitin sulfate or with respect to the total weight of chondroitin sulfate.

According to a preferred aspect of the invention, chondroitin sulfate or a salt thereof (CS) of the present invention (process P1 and/or P3) has a weight average molecular weight comprised from greater than 5 kDa to 10 kDa, and wherein said chondroitin sulfate or the salt thereof comprises:
- a 6-chondroitin sulfate at a weight percentage comprised from 78% to 86%;
- a non-sulfated chondroitin at a weight percentage comprised from 8% to 13%;
- a 2,6-chondroitin disulfate at a weight percentage comprised from 0.3% to 5%; and
- a 4-chondroitin sulfate at a weight percentage comprised from 0.1% to 1.5%, all the percentages being expressed with respect to the total of disaccharides contained in chondroitin sulfate or with respect to the total weight of chondroitin sulfate.

According to a preferred aspect of the invention, chondroitin sulfate or a salt thereof (CS) of the present invention (process P1 and/or P3) has a weight average molecular weight comprised from greater than 5 kDa to less than 50 kDa, preferably from greater than 5 kDa to less than 25 kDa, and wherein said chondroitin sulfate or the salt thereof comprises:
- a 6-chondroitin sulfate at a weight percentage comprised from 50% to 95±0.5%, preferably from 75% to 90%; and
- a 4-chondroitin sulfate at a weight percentage comprised from 0.01% to 5%, preferably from 0.05% to 3%, all the percentages being expressed with respect to the total of disaccharides contained in chondroitin sulfate or with respect to the total weight of chondroitin sulfate.

According to a further preferred aspect of the invention, chondroitin sulfate or a salt thereof (CS) of the present invention (process P1 and/or P3) has a weight average molecular weight comprised from greater than 5 kDa to 10 kDa, and wherein said chondroitin sulfate or the salt thereof comprises:
- a 6-chondroitin sulfate at a weight percentage comprised from 78% to 86%; and
- a 4-chondroitin sulfate at a weight percentage comprised from 0.1% to 1.5%, all the percentages being expressed with respect to the total of disaccharides contained in chondroitin sulfate or with respect to the total weight of chondroitin sulfate.

According to a further preferred aspect of the invention, chondroitin sulfate or a salt thereof (CS) of the present invention (process P1 and/or P3) has a weight average molecular weight comprised from greater than 5 kDa to less than 50 kDa, preferably from greater than 5 kDa to less than 25 kDa, and it comprises:
- a 6-chondroitin sulfate at a weight percentage comprised from 50% to 95±0.5%, preferably from 75% to 90%;
- a non-sulfated chondroitin at a weight percentage comprised from 5% to 20%, preferably from 7% to 15%;
- a 2,6-chondroitin disulfate at a weight percentage comprised from 0.1% to 10%, preferably from 0.2% to 8%;
- a 4-chondroitin sulfate at a weight percentage comprised from 0.01% to 5%, preferably from 0.05% to 3%;
- a 4,6-chondroitin disulfate at a weight percentage comprised from 0.01% to 5%, preferably from 0.05% to 3%; and
- a 2,4-chondroitin disulfate at a weight percentage comprised from 0.01% to 5%, preferably comprised from 0.05% to 3%, all the percentages being expressed with respect to the total of disaccharides contained in chondroitin sulfate or with respect to the total weight of chondroitin sulfate.

According to an embodiment, chondroitin sulfate or a salt thereof (CS) of the present invention (process P1 and/or P3) has a weight average molecular weight comprised from greater than 5 kDa to 10 kDa, and wherein said chondroitin sulfate or the salt thereof comprises:
- a 6-chondroitin sulfate at a weight percentage comprised from 78% to 86%;
- a non-sulfated chondroitin at a weight percentage comprised from 8% to 13%;
- a 2,6-chondroitin disulfate at a weight percentage comprised from 0.3% to 5%; and, furthermore,
- a 4-chondroitin sulfate, 4,6-chondroitin disulfate and 2.4-chondroitin disulfate, each at a weight percentage comprised from 0.1% to 1.5%.

### EXPERIMENTAL PART

I. Process for preparing hyaluronic acid according to the second embodiment (P2).
   (I) prepare dried fungi belonging to the species *Tremella fuciformis,*
   (ii) crush or pulverise said dried fungi (about 20 mesh) by grinding to obtain crushed/pulverised dry fungi;
   (pre-iii) carry out enzymatic hydrolysis, using a pectinase as an enzyme, by adding to the crushed/pulverised dry fungi and to an enzyme pectinase (Pectinex^{®} Ultra Tropical, volume 0.01%) of distilled water (50 volume/weight) and heating at about 50°C for about 3 hours to obtain a hydrolysis mixture;
   (iii. a) carry out a first extraction by adding distilled water (50 volume/weight) to said hydrolysis mixture and heating at about 100°C(boiling) for about 2.5 hours, and filtering with a 200-mesh sieve and collecting a filtrate and a solid residue;
   (iii.b) carry out a second extraction carried out on said solid residue obtained from said first extraction (iii.a) by adding distilled water (50 volume/weight) and heating to about 100°C (boiling) for about 2 hours, followed by filtering with 200 mesh sieve and collecting a filtrate; and combine the filtrate obtained from said first extraction (iii.a) with the filtrate obtained from said second extraction (iii.b) to obtain an aqueous extract;
   (iii. c) concentrate said aqueous extract at about 75°C to obtain a concentrated aqueous extract having a relative density of about 1.05; followed by
   (iv) slowly adding, under stirring, to said concentrated aqueous extract, 95% ethanol (volume/volume = 3) and leaving for 12 hours; remove the ethanol and keep the extract;
   (vii) add distilled water to said extract and freeze-dry to obtain a product PR1 comprising or, alternatively, consisting of hyaluronic acid or a salt thereof (HA) having an average molecular weight of from 100 to 300 kDa and a purity by weight percentage comprised from 95% to 99% with respect to the total weight of said product PR1.
II. Process for preparing chondroitin sulfate according to the third embodiment (P3).
   (i) prepare dried fungi belonging to the species *Tremella fuciformis;*
   (ii) crush or pulverise the dried fungi (20 mesh) by grinding to obtain crushed/pulverised dry fungi;
   (pre-iii) carry out enzymatic hydrolysis, using a pectinase as an enzyme, by adding to the crushed/pulverised dry fungi and to an enzyme pectinase (Pectinex^{®} Ultra Tropical, volume 0.01%) of distilled water (50 volume/weight) and heating at about 50°C for about 2 hours to obtain a hydrolysis mixture (pH from 5-7);
   (iii.a) carry out a first extraction by adding distilled water (50 volume/weight) to the hydrolysis mixture and heating at 100°C (boiling) for about 2.5 hours, followed by filtration with a 200-mesh sieve (if necessary centrifuging before filtering) and collecting a filtrate and a solid residue;
   (iii.b) carry out a second extraction carried out of said solid residue obtained from said first extraction by adding distilled water (50 volume/weight) and heating at about 100°C (boiling) for about 1.5 hours, followed by filtering with 200 mesh sieve and collecting a filtrate; and combine the filtrate obtained from said first extraction (iii.a) with said filtrate obtained from said second extraction (iii.b) to obtain an aqueous extract;
   (iii. c) concentrate said aqueous extract at about 75°C to obtain a concentrated aqueous extract having a relative density of about 1.05-1.08; followed by
   (vi.c) carry out a sulfonation (or sulfation) reaction by adding SO₃ DMF 5 ml, 15 ml, 55 ml, for every 100 ml of concentrated aqueous extract, at about 75°C for about 30 minutes;
   (vi.d) neutralise with NaOH up to about a pH value of 7;
   (vi.e) place the obtained solution in a dialysis bag (1,000 Da) for at least 24 hours, until a relative density of the solution of about 1.1 is reached. Dry in a vacuum oven to obtain a product PR2 comprising or, alternatively, consisting of chondroitin sulfate or a salt thereof (CS) having an average molecular weight comprised from greater than 5 kDa to 10 kDa (for example, about 8 kDa), wherein said CS has a composition similar to the compound CS.1 reported in Table 1 and a purity at a weight percentage comprised from 89% to 94.5%, with respect to the total weight of said product PR2.

## Claims

1. A process for preparing a mixture comprising or, alternatively, consisting of at least one glycosaminoglycan selected from a chondroitin sulfate or a salt thereof (CS), said process comprising at least one step for extracting from a starting material of plant origin with an aqueous solvent or water, wherein said starting material of plant origin comprises or, alternatively, consists of at least one natural fungus belonging to the *Dikarya* subkingdom, *Basidiomycota* division, and to the species *Tremella fuciformis* (Berk. 1856).

2. The process according to claim 1, comprising the following steps:
(i) identifying said at least one natural fungus as a plant starting material of said at least one glycosaminoglycan;
(ii) optionally, carrying out a crushing or pulverisation of the plant starting material to obtain said at least one pulverised and crushed fungus;
(iii) carrying out an extraction of said glycosaminoglycan from said at least one fungus obtained from step (i) or from step (ii) using an extraction solvent, preferably an aqueous solvent, even more preferably water to obtain an aqueous extract of said glycosaminoglycan;
(iv) adding a solvent, preferably an alcohol, more preferably ethanol to the aqueous extract obtained from step (iii) to obtain a liquid product;
(v) carrying out a centrifugation and/or a filtration of said liquid product obtained from step (iv) to obtain a liquid phase and a solid residue;
(vi) carrying out a processing of said solid residue obtained by centrifugation and/or filtration of step (v) through the following steps (vi.b), (vi.c), (vi.d) and (vi.e):
(vi.b) recovering and purifying said solid residue obtained from step (v) to obtain a product of step (vi.b);
(vi.c) treating said product of step (vi.b) with a source of sulfuric acid, preferably wherein said source of sulfuric acid is selected from the group comprising or, alternatively, consisting of: sulfuric acid, a sulfur-trioxide pyridine complex, a sulfur trioxide dimethyl-formamide complex and mixtures thereof, more preferably SO₃-DMF, to obtain an acidified product;
(vi.d) neutralising said acidified product obtained from step (vi.c) using a basic agent, preferably sodium hydroxide, to obtain a neutralised product;
(vi.e) concentrating and drying said neutralised product obtained from step (vi.d) to obtain a product PR2 comprising or, alternatively, consisting of chondroitin sulfate or a salt thereof having a weight average molecular weight comprised from 1 kDa to 50 kDa.

3. The process according to claim 2, wherein the step (iii) for the extraction of said at least one glycosaminoglycan from the plant starting material comprises or, alternatively, consists of the following steps:
(iii.a) carrying out a first extraction from the plant starting material with a first volume of extraction solvent at a temperature comprised from 10°C to 90°C for a period of time comprised from 0.5 hours to 12 hours to obtain first aqueous extract and a solid residue; and
(iii.b) carrying out a second extraction of said solid residue obtained from step (iii.a) with a second volume of extraction solvent at a temperature comprised from 90°C to 110°C, for a period of time comprised from 0.5 hours to 8 hours, to obtain a second aqueous extract; and combining said first extract and said second extract to obtain an aqueous extract.

4. The process according to claim 3, wherein in the step (iii) of carrying out an extraction comprising or, alternatively, consisting of said step (iii.a) of carrying out a first extraction and said step (iii.b) of carrying out a second extraction, an enzyme, preferably a proteolytic enzyme, is used.

5. The process according to any one of claims 1-4, wherein said chondroitin sulfate, or the salt thereof, obtained from the process has a weight average molecular weight comprised from 5 kDa or greater than 5 kDa to 25 kDa; preferably from 5 kDa or greater than 5 kDa to 10 kDa; and/or
wherein said chondroitin sulfate, or the salt thereof, obtained from the process comprises or, alternatively, consists of 6-chondroitin sulfate at a % by weight comprised from 50% to 95±0.5%, with respect to the total of disaccharides contained in chondroitin sulfate or salt thereof.

6. Use of a fungus belonging to the *Dikarya* subkingdom, preferably to the *Basidiomycota* division, more preferably to the species *Tremella fuciformis* (Berk. 1856), as a plant starting material for preparing a chondroitin sulfate or a salt thereof having a weight average molecular weight comprised from 1 kDa to 50 kDa; preferably from 5 kDa or greater than 5kDa to 25 kDa; more preferably from 5 kDa or greater than 5kDa to 10 kDa.

7. Use of a fungus belonging to the *Dikarya* subkingdom, preferably to the *Basidiomycota* division, more preferably to the species *Tremella fuciformis* (Berk. 1856), as a plant starting material for preparing a chondroitin sulfate or a salt thereof according to claim 6, wherein said chondroitin sulfate or said salt thereof comprises or, alternatively, consists of 6-chondroitin sulfate at a % by weight comprised from 50% to 95±0.5%, with respect to the total of disaccharides contained in chondroitin sulfate or salt thereof.

8. A chondroitin sulfate or a salt thereof, having a weight average molecular weight comprised from 1 kDa to less than 50 kDa, preferably comprised from 5 kDa to greater than 5 kDa to 25 kDa or from greater than 5 kDa to 10 kDa.

9. The chondroitin sulfate or a salt thereof according to claim 8, wherein said chondroitin sulfate or a salt thereof comprises 6-chondroitin sulfate at a % by weight comprised from 50% to 95%, the % being expressed with respect to the total of disaccharides contained in chondroitin sulfate.

10. The chondroitin sulfate or salt thereof (CS) according to claims 8 and 9 having a weight average molecular weight comprised from greater than 5 kDa to 25 kDa, preferably greater than 5 kDa to 10 kDa, and wherein said chondroitin sulfate or the salt thereof comprises: 6-chondroitin sulfate at a % by weight comprised from 75% to 90%, preferably from 78% to 86%, and 4-chondroitin sulfate at a % by weight comprised from 0.05% to 3%, preferably from 0.1% to 1.5%, the % being expressed with respect to the total of disaccharides contained in chondroitin sulfate.

11. Use of the chondroitin sulfate or a salt thereof according any one of claims 8-10 as additive or excipient, or ingredient in the preparation of pharmaceutical products, medical devices, nutraceutical products, food for special medical purposes (SFMPs), dietary supplements or food products.

12. A composition comprising: (i) the chondroitin sulfate or a salt thereof according to any one of claims 8-10, and (ii) technological additives or pharmaceutical or food grade excipients.

13. The composition according to the preceding claim for use as medicament.

14. The composition according to claim 13 for use in a method for preventive and/or curative treatment of arthritis, osteoarthritis, arthrosis, joint pain, inflammation of the limbs and of the joints, or gastroesophageal reflux, in human or animal subjects.
